(19) Europäisches Patentamt
European Patent Office
Office européen des brevets

(11) EP 0 634 411 B1

(12) **EUROPÄISCHE PATENTSCHRIFT**

(45) Veröffentlichungstag und Bekanntmachung des Hinweises auf die Patenterteilung:
**26.05.1999 Patentblatt 1999/21**

(51) Int Cl.[6]: **C07D 401/12**

(21) Anmeldenummer: **94111037.1**

(22) Anmeldetag: **15.07.1994**

(54) **Verfahren zur Herstellung von 8- 4'-[4"-(Pyrimidin-2'"-yl)-piperazin-1"-yl]-butyl -8-aza-spiro[4,5] decan-7,9-dion (= Buspiron) und dessen Hydrochloriden in hoher Reinheit**

Process for the preparation of 8- 4'-[4"-(pyrimidin-2'"-yl)-piperazin-1"-yl]-butyl -8-aza-spiro[4,5]decan-7,9-dione and of its hydrochlorides of high purity

Procédé de préparation de 8- 4'-[4"-(pyrimidin-2'"-yle)-piperazin-1"-yle]-butyle -8-aza-spiro[4,5]decan-7,9-dione et leur hydrochlorides de haute pureté

(84) Benannte Vertragsstaaten:
**AT BE CH DE DK LI NL SE**

(30) Priorität: **16.07.1993 HU 9302040**

(43) Veröffentlichungstag der Anmeldung:
**18.01.1995 Patentblatt 1995/03**

(73) Patentinhaber: **EGIS GYOGYSZERGYAR RT.
1106 Budapest (HU)**

(72) Erfinder:
- **Dr. Tibor Mezei, Chimieingenieur
H 1221 Budapest (HU)**
- **Dr. Gabor Blasko, Chemieingenieur
H 1113 Budapest (HU)**
- **Dr. Zoltan Budai, Chemieingenieur
H 1023 Budapest (HU)**
- **Dr. Margit Csörgo, Ärztin
H 1174 Budapest (HU)**
- **Dr. Eva Furdyga, Chemieingenieurin
H 1013 Budapest (HU)**
- **Dr. Imre Klebovich, Pharmazeut
H 1125 Budapest (HU)**
- **Laszlo Koncz, Chemieingenieur
Mogyorod (HU)**
- **Dr. Ilona Szaruhar, Chemieingenieurin
H 1191 Budapest (HU)**
- **Dr. Attila Mandi, Chemieingenieur
H 1026 Budapest (HU)**
- **Dr. Kalman Nagy, Chemieingenieur
H 1022 Budapest (HU)**
- **Dr. Klara Reiter geb. Esses, Chemieingenieurin
H 1022 Budapest (HU)**
- **Dr. Gyula Simig, Chemieingenieur
H 1126 Budapest (HU)**
- **Dr. Judit Szego, Ärztin
H 1036 Budapest (HU)**
- **Gyöngyi Vereszkey geb. Donath, Chemieingenieurin
H 1034 Budapest (HU)**

(74) Vertreter: **Beszédes, Stephan G., Dr.
Patentanwalt
Postfach 11 68
85201 Dachau (DE)**

(56) Entgegenhaltungen:
**EP-A- 0 304 940 EP-A- 0 304 941
DE-A- 3 806 009**

- **DATABASE WPI Week 8808, Derwent Publications Ltd., London, GB; AN 88-053931 & CHEMICAL ABSTRACTS, vol. 110, no. 3, 16. Januar 1989, Columbus, Ohio, US; abstract no. 23914e, KOJIMA Y ET AL. 'Preparation of N-(piperazinylbutyl)imide derivatives as tranquilizers and psychotropics' Seite 557 ;Spalte 1 ; & JP-A-63 010 760 (SUMITOMO PHARMACEUTICALS CO.,LTD.) 18. Januar 1988**
- **CHEMICAL ABSTRACTS, vol. 110, no. 25, 19. Juni 1989, Columbus, Ohio, US; abstract no. 231663z, KOJIMA Y ET AL. 'Preparation of (piperazinylbutynyl)- and (piperazinylbutenyl)bicycloheptane derivatives as tranquilizers' Seite 652 ;Spalte 1 ; & JP-A-63 010 786 (SUMITOMO PHARMACEUTICALS CO.,LTD.) 18. Januar 1988**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 634 411 B1

- CHEMICAL & PHARMACEUTICAL BULLETIN Bd. 39, Nr. 9 , September 1991 Seiten 2288 - 2300 ISHIZUMI K. ET AL. 'Synthesis and anxiolytic activity of N-substituted cyclic imides (1R,2S,3R,4S)-N-[4-[4-(2-pyr imidinyl)-1-piperazinyl]butyl]-2,3-bicyclo [2.2.1]heptanedicarboximide (Tandospirone) and related compounds'
- SCIENTIA PHARMACEUTICA Bd. 58 , 1990 Seiten 37 - 53 KUHNERT-BRANDSTÄTTER M. & PORSCHE U. 'Beitrag zur Polymorphie von Arzneistoffen. 6. Mitteilung: Bupicomid, Buspironhydrochlorid, Heptolamid, Levobunololhydrochlorid, Naftifinhydrochlorid und Piretanid'

## Beschreibung

Die Erfindung betrifft ein neues, besseres Verfahren zur Herstellung von 8-{4'-[4"-(Pyrimidin-2'"-yl)-piperazin-1"-yl]-butyl}-8-aza-spiro[4,5]decan-7,9-dion und von dessen Hydrochloriden hoher Reinheit frei von Nebenprodukten.

8-{4'-[4"-(Pyrimidin-2'"-yl)-piperazin-1"-yl]-butyl}-8-aza-spiro[4,5]decan-7,9-dion der Formel

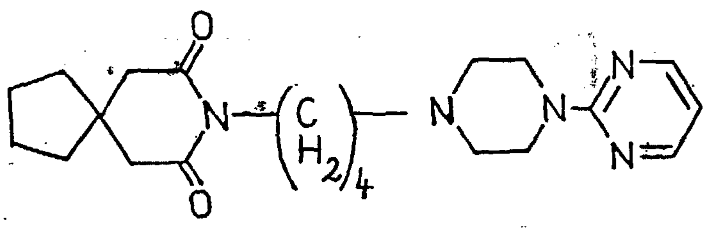

I

ist ein bekannter wertvoller anxioselektiver Wirkstoff (GB-PS 1 332 194).

Zur Herstellung dieser Verbindung sind mehrere Verfahren bekannt.

Nach der GB-PS 1 332 194 wird das 8-{4'-[4"-(Pyrimidin-2'"-yl)-piperazin-1"-yl]-butyl}-8-aza-spiro[4,5]decan-7,9-dion der Formel I in der Weise hergestellt, daß 8-Oxa-spiro[4,5]decan-7,9-dion mit 1-[4'-(Amino)-butyl]-4-[pyrimidin-2"-yl]-piperazin umgesetzt wird. Die Umsetzung wird in Pyridin beim Siedepunkt des Reaktionsgemisches durchgeführt. Das gewünschte 8-{4'-[4"-(Pyrimidin-2'"-yl)-piperazin-1"-yl]-butyl}-8-aza-spiro[4,5]decan-7,9-dion der Formel I wird in Form eines Rohproduktes in mittelmäßigen Ausbeuten erhalten. Das Rohprodukt wird in Form der freien Base entweder durch Kristallisation oder durch fraktionierte Vakuumdestillation gereinigt. Der Nachteil der erstgenannten Reinigungsverfahrensweise liegt in den hohen Verlusten. Die fraktionierte Destillation wird wiederum bei hoher Temperatur (240 bis 265°C) unter niedrigem Druck (13,3 Pa) durchgeführt und stellt eine schwere thermische Belastung dar und führt daher zu einer Schädigung des Produktes.

Nach einem in der GB-PS 1 332 194 beschriebenen anderen Verfahren wird 8-[4'-(Chlor)-butyl]-8-aza-spiro [4,5]decan-7,9-dion mit N-[Pyrimidin-2'-yl]-piperazin in Gegenwart von Natriumcarbonat in n-Butanol beim Siedepunkt des Reaktionsgemisches 3 Tage lang umgesetzt. Die äußerst lange Reaktionszeit schließt eine wirtschaftliche Durchführung dieses Verfahrens im Betriebsmaßstab aus. Ein weiterer Nachteil besteht darin, daß die Reinigung des Produktes sehr kompliziert und mit hohem Aufwand verbunden ist sowie mit großer Sorgfalt durchgeführt werden muß. Ein noch weiterer Nachteil des Verfahrens liegt darin, daß das bei der Herstellung des 8-[4'-(Chlor)-butyl]-8-aza-spiro [4,5]decan-7,9-diones verwendete 1-(Brom)-4-(chlor)-butan eine sehr schwer zugängliche und nur schwerfällig herstellbare Verbindung ist.

Nach einem in der GB-PS 1 332 194 beschriebenen weiteren Verfahren wird das aus 1-(Brom)-4-(chlor)-butan hergestellte 1-[4'-(Chlor)-butyl]-4-[pyrimidin-2'-yl]-piperazin mit 8-Aza-spiro-[4,5]decan-7,9-dion umgesetzt. Das Verfahren wird in analoger Weise wie im vorherigen Absatz beschrieben durchgeführt. Dieser Syntheseweg enthält mehrere empfindliche, chemisch nur schwer durchführbare Stufen. Das gewünschte Endprodukt 8-{4'-[4"-(Pyrimidin-2'"-yl)-piperazin-1"-yl]-butyl}-8-aza-spiro[4,5-decan-7,9-dion der Formel I kann in zur pharmazeutischen Anwendung geeigneter Form nur nach mehreren Reinigungsstufen erhalten werden. Ein weiterer Nachteil besteht in der schweren Zugänglichkeit des als Ausgangsstoff eingesetzten 1-(Brom)-4-(chlor)-butans.

Das 1-[4'-(Amino)-butyl]-4-[pyrimidin-2"-yl]-piperazin kann in der Weise hergestellt werden, daß 1-[Pyrimidin-2'-yl]-piperazin mit 3-(Chlor)-propionitril in n-Butanol als Medium beim Siedepunkt des Reaktionsgemisches während einer längeren Zeit (etwa 16 Stunden) erhitzt wird. Das erhaltene Zwischenprodukt muß durch Kristallisieren gereinigt werden (Ausbeute 70%). Das als Zwischenprodukt erhaltene Nitril wird in einer Ausbeute von etwa 70% katalytisch hydriert (GB-PS 1 332 194).

Nach der ungarischen Patentschrift 187 999 wird das 8-{4'-[4"-(Pyrimidin-2'"-yl)-piperazin-1"-yl]-butyl}-8-aza-spiro[4,5]decan-7,9-dion der Formel I wie folgt hergestellt: Aus 1-[4'-(Chlor)-butyl]-4-[pyrimidin-2'-yl]-piperazin wird zunächst das spiro-quaternäre Ammonium-piperazin-Derivat der Formel

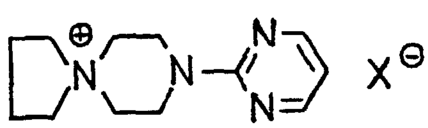

III

gebildet, welches in Gegenwart einer starken Base mit 8-Aza-spiro[4,5]decan-7,9-dion umgesetzt wird. Dieses Verfahren ist mit schwerwiegenden Nachteilen verbunden. Die Ausbeute ist schlecht, die Synthese besteht aus einer großen Zahl von Stufen und die Reinigung des Produktes stößt auf Schwierigkeiten, weswegen die Durchführung im Betriebsmaßstab nicht in Betracht kommt.

Nach der schweizerischen Patentschrift 647 518 wird das 8-Aza-spiro[4,5]decan-7,9-dion zunächst mit 1,4-Di-(brom)-butan umgesetzt, worauf das erhaltene 4-(Brom)-butyl-Derivat mit Piperazin umgesetzt und das erhaltene Produkt schließlich mit 2-(Chlor)-pyrimidin zur Umsetzung gebracht wird. Das Ziel dieses Verfahrens ist die Herstellung der mit dem $^{14}C$-Isotop markierten Verbindung und das Verfahren ist zur Verwirklichung im Betriebsmaßstab ungeeignet.

Nach der spanischen Patentschrift 536 286 wird das Kaliumsalz des 8-Aza-spiro[4,5]-decan-7,9-diones mit 4-(Chlor)-butyraldehyd umgesetzt, worauf das erhaltene Produkt unter reduktiven Bedingungen mit N-[Pyrimidin-2-yl]-piperazin umgesetzt wird. Dieses Verfahren ist nur von theoretischer Bedeutung und spielt bei der Herstellung im Betriebsmaßstab keine Rolle.

In der DOS 3 806 009 wird das Ziel gesetzt, die obigen Nachteile der bekannten Verfahren zu beheben. Das Wesen des dort beschriebenen Verfahrens liegt darin, daß das 8-{4'-[4"-(Pyrimidin-2'''-yl)-piperazin-1"-yl]-but-2'-inyl}-8-aza-spiro[4,5]decan-7,9-dion durch katalytisches Hydrieren in 8-{4'-[4"-(Pyrimidin-2'''-yl)-piperazin-1"-yl]-butyl}-8-aza-spiro[4,5]decan-7,9-dion überführt wird.

Das Hydrieren wird in Gegenwart eines Metallkatalysators, vorteilhaft von Palladium, vorzugsweise auf Kohle aufgebrachtem Palladium, in einem organischen Lösungsmittel durchgeführt. Als Reaktionsmedium werden aliphatische Alkohole verwendet. Der Schmelzpunkt des so hergestellten Produktes beträgt 91 bis 99°C.

Eine stürmische Entwicklung der zur Bestimmung der Reinheit dienenden Apparate und Verfahren sowie die schnelle Erhöhung der Anforderungen machen es zu einer zwingenden Notwendigkeit, immer reinere Substanzen zur Verwendung als Arzneimittel herzustellen.

Nach den Vorschriften der modernen Pharmakopöe ist die obere Grenze sämtlicher vorliegenden Verunreinigungen im allgemeinen mit 0,5 Gew.-% festgesetzt, wobei die Menge der nicht identifizierbaren Verunreinigungen den Wert von 0,1 Gew.-% nicht übersteigen darf.

Nach eigenen Versuchen kann 8-{4'-[4"-(Pyrimidin-2'''-yl)-piperazin-1"-yl]-butyl}-8-aza-spiro[4,5]decan-7,9-dion höchster Reinheit durch das in der DOS 3 806 009 beschriebene Verfahren nicht hergestellt werden. Das nach dieser DOS 3 806 009 hergestellte 8-{4'-[4"-(Pyrimidin-2'''-yl)-piperazin-1"-yl]-butyl}-8-aza-spiro[4,5]decan-7,9-dion enthält mehrere Verunreinigungen. Die Bestimmung der Struktur dieser Substanzen zeigte, daß die Verunreinigungen einerseits von der unvollständigen katalytischen Hydrierung herstammen und die ungesättigten Verbindungen, das heißt 8-{4'-[4"-(Pyrimidin-2'''-yl)-piperazin-1"-yl]-but-2'-inyl}-8-aza-spiro[4,5]decan-7,9-dion und 8-{4'-[4"-(Pyrimidin-2'''-yl)-piperazin-1"-yl]-but-2'-enyl}-8-aza-spiro[4,5]decan-7,9-dion das Endprodukt verunreinigen und andererseits nach der Hydrierung entstehen. Die Struktur der letzteren Verbindungen wurde durch GC/MS-Verfahren bestimmt und als 1-[Pyrimidin-2'-yl]-piperazin und 8-[Aza]-spiro[4,5]decan-8-[butyl]-7,9-dion identifiziert. Das Entstehen dieser Verunreinigungen läßt sich mit dem Artikel von G. F. Hennion und G. A. Ferrino (J. Org. Chem. 26 [1961], 1 073) gut erklären; nach dieser Literaturstelle kann nach der partiellen Sättigung der Dreifachbindung die C-N-Bindung gespalten werden.

Ferner ist aus JP-A-63-10760 ein Verfahren unter anderen auch von 8-{4'-[4"-(Pyrimidin-2'''-yl)-piperazin-1"-yl]--butyl}-8-aza-spiro[4,5]decan-7,9-dion durch Hydrieren von 8--{4'-[4"-(Pyrimidin-2'''-yl)-piperazin-1"-yl]-but-2'inyl}-8--aza-spiro[4,5]decan-7,9-dion mit Wasserstoff in Gegenwart eines Palladium-Katalysators bekannt. Dazu wird nach den Ausführungsbeispielen zu einer Tetrahydrofuran-Lösung (gemeint ist wohl eine Suspension) eines in einem Wasserstoffgasstrom vorbehandelten Palladium-Katalysators eine Tetrahydrofuran-Lösung von 8-{4'-[4"-(Pyrimidin-2'''-yl-piperazin-1"-yl]--but-2'-inyl}-8-aza-spiro[4,5]decan-7,9-dion unter Rühren zugegeben. Von einer kontinuierlichen Zugabe ist keine Rede. Vielmehr wird nach dem die Umsetzung der der obigen Ausgangssubstanz ähnlichsten Ausgangssubstanz zum dem obigen Endprodukt ähnlichsten Endprodukt betreffenden "Working Example 4" die Hydrierung unter Rühren unter 8 Atm 5 Stunden lang durchgeführt (im "Working Example 5" sind 4 Stunden angegeben). Daraus geht hervor, daß die Ausgangssubstanz schnell, auf einmal zugesetzt wird. Im "Working Example 4" beträgt die Konzentration des 8-{4'-[4"-(Pyrimidin-2'''-yl)-piperazin-1"--yl]-but-2-inyl}-8-aza-spiro[4,5]decan-7,9-diones nur 12,7% (im "Working Example 5" ist sie nur 2,5%) und der Konzentrationsbereich der Ausgangssubstanzen erstreckt sich in den "Working Examples" von 2,5 bis 18,9%. Die Qualität beziehungsweise Reinheit der erhaltenen Produkte betreffend wird in der genannten Druckschrift nur ein Schmelzpunkt von 204 bis 205°C behauptet, was aber erst nach einer säulenchromatographischen Reinigung erreicht wird. Die Ausbeute beträgt nur 82%.

Ferner ist aus Chem. Pharm. Bull. 39 (9) [1991], Seiten 2 288 bis 2 300 das Hydrieren von rohem 8-{4'-[4"-(Pyrimidin--2'''-yl)-piperazin-1"-yl]-but-2'-inyl}-8-aza-spiro[4,5]decan--7,9-dion in Tetrahydrofuran über 10% Palladium auf Kohle zu 8-{4'-[4"-(Pyrimidin-2'''-yl)-piperazin-1"-yl]-butyl}-8--aza-spiro[4,5]decan-7,9-dion bekannt. Die Konzentration der genannten Ausgangssubstanz in Tetrahydrofuran beträgt ausweislich des Beispieles auf Seite 2 299, Absatz 4 nur 2%. Auch ist keine Rede von einer Zugabe der Lösung der genannten Ausgangssubstanz zur Suspension des Katalysators in Tetrahydrofuran, geschweige denn von einer kontinuierlichen. Für das erhaltene Produkt ist der verhältnismäßig niedrige Schmelzpunkt von 200 bis 202°C angegeben.

Außerdem ist in Chemical Abstracts, Vol. 110, No. 25, 19. Juni 1989, Abstract No. 231 663z (Referat von JP-A--63 10, 786) die Umsetzung einer Mischung von exo-N-Propargylbicyclo[2,2,1]heptan-2,3-dicarboximid, 1-(2-pyrimidinyl)--piperazin und von 35%-igem Formaldehyd in Dioxan mit Kupfersulfat durch 2 Stunden langes Rühren bei 70 bis 80°C zu exo-N-[4-{4-(2-pyrimidinyl)-1-piperazinyl}-2-butinyl]-bicyclo[2,2,1]heptan-2,3-dicarboximid beschrieben.

Es ist bekannt, daß das Hydrochloridsalz des 8-{4'-[4''-(Pyrimidin-2'''-yl)-piperazin-1''-yl]-butyl}-8-aza-spiro [4,5]decan-7,9-diones in verschiedenen Kristallformen kristallisierbar ist und die verschiedenen Polymorphe, nämlich das mit einem Schwelzpunkt von 188 bis 191°C (in folgenden P 188 genannt) und das mit einen Schmelzpunkt von 201 bis 203°C (im folgenden P 203 genannt), ineinander überfürbar sind. Dieses Produkt ist in der US-PS 4 810 789 offenbart. Die beiden Polymorphe sind durch ihre Schmelzpunkte, welche durch DSC (Differential Scanning Calorimetry) gemessen werden, charakterisiert.

Ferner ist bekannt, daß das IR-Spektrum der verschiedenen Polymorphe minimale Abweichungen (Unterschiede) zeigt (M. Kuhnert-Brandstätter, U. Porsche: Scientia Pharmaceutica 58 [1990], 37 bis 53). Das Polymorph P 188 zeigt eine Absorption bei 1 290 $cm^{-1}$ und das Polymorph P 203 zeigt eine Absorption bei 1 150 $cm^{-1}$. Die Ab- beziehungsweise Anwesenheit dieser charakteristischen Absorption beweist die Gegenwart des einen oder des anderen Polymorphes und das Verhältnis der Intensitäten der beiden Signale weist auf das Verhältnis der Polymorphe hin.

Nach der EP 304 940 A1 wird das 8-{4'-[4''-(Pyrimidin-2'''-yl)-piperazin-1''-yl]-butyl}-8-aza-spiro[4,5]decan-7,9-dion-hydrochlorid P 203 in der Weise hergestellt, daß die Kristallstruktur der Form P 188 oder einer Mischung der Formen P 188 und P 203 des 8-{4'-[4''-(Pyrimidin-2'''-yl)-piperazin-1''-yl]-butyl}-8-aza-spiro[4,5]decan-7,9-dion-hydrochlorides durch partielles oder vollständiges Auflösen zerstört wird, die Verbindung bei einer Temperatur über 95°C auskristallisiert wird und die ausgeschiedenen Kristalle abgetrennt werden. Als Lösungsmittel werden vorteilhaft Butanol, Cyclohexanon, Nonan, Xylol oder deren Gemische verwendet. Nach der EP 304 941A1 wird das Polymorph P 188 des 8-{4'-[4''-(Pyrimidin-2'''-yl)-piperazin-1''-yl]-butyl}-8-aza-spiro[4,5]decan-7,9-dion-hydrochlorides in der Weise hergestellt, daß die Kristallstruktur der Form P 203 des 8-{4'-[4''-(Pyrimidin-2'''-yl)-piperazin-1''-yl]-butyl}-8-aza-spiro [4,5]decan-7,9-dion-hydrochlorides durch partielles oder vollständiges Auflösen zerstört wird, die Verbindung bei einer unter 95°C liegenden Temperatur auskristallisiert wird und die ausgeschiedenen Kristalle abgetrennt werden.

Nach den EP 304 940 A1 und 304 941 A1 wird beim Umkristallisieren des 8-{4'-[4''-(Pyrimidin-2'''-yl)-piperazin-1''-yl]-butyl}-8-aza-spiro[4,5]decan-7,9-dion-hydrochlorides dem Temperaturgrenzwert von 95°C eine sehr große Bedeutung beigemessen. Gemäß diesen europäischen Patentanmeldungen kristallisiert bei einer Temperatur unter 95°C das Polymorph P 188 und bei einer über 95°C liegenden Temperatur das Polymorph P 203 des 8-{4'-[4''-(Pyrimidin-2'''-yl)-piperazin-1''-yl]-butyl}-8-aza-spiro[4,5]decan-7,9-dion-hydrochlorides aus. In diesen Patentanmeldungen werden über die Herstellung der 8-{4'-[4''-(Pyrimidin-2'''-yl)-piperazin-1''-yl]-butyl}-8-aza-spiro[4,5]decan-7,9-dion-Base keine ausführlichen Informationen gegeben.

Der Erfindung liegt die Aufgabe zugrunde, unter Behebung der Nachteile der bekannten Verfahren, insbesondere des in der DOS 3 806 009 beschriebenen Verfahrens, ein Verfahren zur Herstellung von 8-{4'-[4''-(Pyrimidin-2'''-yl)-piperazin-1''-yl]-butyl}-8-aza-spiro[4,5]decan-7,9-dion und von dessen Hydrochloriden hoher Reinheit, durch welches den Forderungen der Pharmakopöe entsprechendes hochreines 8-{4'-[4''-(Pyrimidin-2'''-yl)-piperazin-1''-yl]-butyl}-8-aza-spiro[4,5]decan-7,9-dion-hydrochlorid unmittelbar, ohne nachträgliche Reinigung hergestellt werden kann, zu schaffen.

Das Obige wurde überraschenderweise durch die Erfindung erreicht.

Die Erfindung beruht auf der überraschenden Feststellung, daß, wenn beim Hydrieren eine Lösung von 8-{4'-[4''-(Pyrimidin-2'''-yl)-piperazin-1''-yl]-but-2'-inyl}-8-aza-spiro[4,5]decan-7,9-dion der Formel

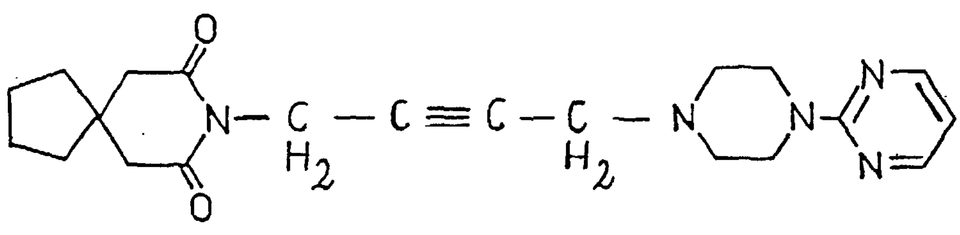

II

in einem inerten organischen Lösungsmittel der Suspension des Katalysators in einem inerten organischen Lösungsmittel zugesetzt wird, hochreines 8-{4'-[4''-(Pyrimidin-2'''-yl)-piperazin-1''-yl]-butyl}-8-aza-spiro[4,5]decan-7,9-dion der Formel

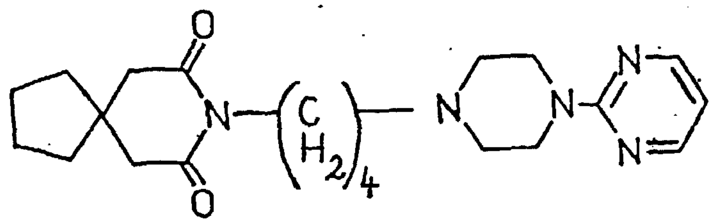

I

isoliert werden kann, welches nach der HPLC-Analyse höchstens 0,1 Gew.-% Verunreinigungen enthält. Dagegen wurde durch Versuche festgestellt, daß die Änderung der Temperatur (zwischen 20 und 60°C) und des Druckes (zwischen 1 und 8 bar) die Reinheit des Produktes und die Ausbeute nicht wesentlich beeinflußt.

Gegenstand der Erfindung ist ein Verfahren zur Herstellung von 8-{4'-[4"-(Pyrimidin-2'"-yl)-piperazin-1"--yl]-butyl}-8-aza-spiro[4,5]decan-7,9-dion {Buspiron} der Formel

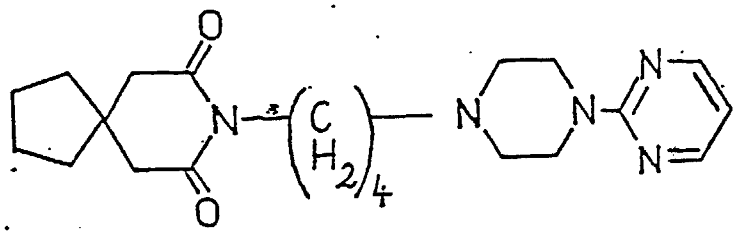

I

und von dessen Hydrochloriden hoher Reinheit durch katalytisches Hydrieren von 8-{4'-[4"-(Pyrimidin-2'"-yl)-piperazin-1"-yl]-but-2'-inyl}-8-aza-spiro[4,5]decan-7,9--dion der Formel

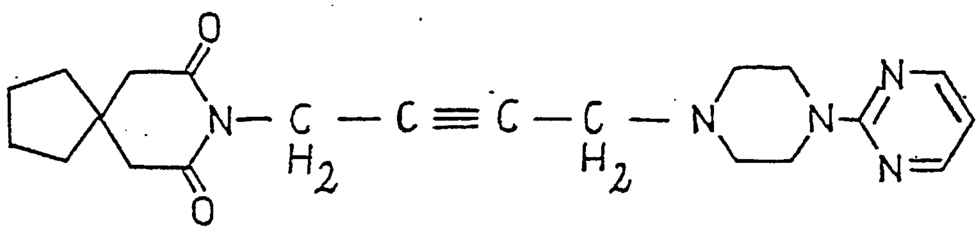

II

in Gegenwart eines Palladium- oder Raney-Nickel-Katalysators in einem inerten organischen Lösungsmittel und gegebenenfalls darauffolgendes Überführen des erhaltenen 8-{4'-[4"-(Pyrimidin-2'"-yl)-piperazin-1"-yl]-butyl}--8-aza-spiro[4,5]decan-7,9-diones in ein Hydrochlorid, welches dadurch gekennzeichnet ist, daß eine Lösung von 8-{4'-[4"-(Pyrimidin-2'"-yl)-piperazin-1"-yl]-but-2'-inyl}--8-aza-spiro[4,5]decan-7,9-dion der Formel II in einem inerten organischen Lösungsmittel, deren Konzentration mindestens 40 Gew./Gew.-% ist, einer Suspension des Katalysators in einem inerten organischen Lösungsmittel kontinuierlich zugesetzt wird, der Katalysator entfernt wird und danach

a) die 8-{4'-[4"-(Pyrimidin-2'"-yl)-piperazin-1"-yl]-butyl}-8-aza-spiro[4,5]decan-7,9-dion-Base der Formel I isoliert wird und/oder

b) die 8-{4'-[4"-(Pyrimidin-2'"-yl)-piperazin-1"-yl]-butyl}-8-aza-spiro[4,5]decan-7,9-dion-Base der Formel I bei einer Temperatur von 15 bis 40°C in Äthanol oder Isopropanol mit Chlorwasserstoff unter Rühren behandelt und das bei 188 bis 191°C schmelzende 8-{4'-[4"-(Pyrimidin-2'"-yl)-piperazin-1"-yl]-butyl}-8-aza-spiro[4,5]decan-7,9-dion-hydrochlorid ⟨Buspironhydrochlorid⟩ isoliert wird oder

c) die 8-{4'-[4"-(Pyrimidin-2'"-yl)-piperazin-1"-yl]-butyl}-8-aza-spiro[4,5]decan-7,9-dion-Base der Formel I bei einer Temperatur von höchstens 70°C in Äthylacetat oder Isopropanol mit Chlorwasserstoff unter Rühren behandelt und das bei 201 bis 203°C schmelzende 8-{4'-[4"-(Pyrimidin-2'"-yl)-piperazin-1"-yl]-butyl}-8-aza-spiro[4,5]decan-7,9-dion-hydrochlorid ⟨Buspironhydrochloric⟩ isoliert wird.

Vorteilhaft wird eine Lösung mit einer Konzentration von 40 bis 70 Gew./Gew.-% am 8-{4'-[4"-(Pyrimidin-2'"-

yl)-piperazin-1''-yl]-but-2'-inyl}-8-aza-spiro[4,5]decan-7,9-dion der Formel II verwendet.

Es ist auch zweckmäßig, für die Lösung des 8-{4'-[4''-(Pyrimidin-2'''-yl)-piperazin-1''-yl]-but-2'-inyl}-8-aza-spiro[4,5]decan-7,9-diones der Formel II als Lösungsmittel ein polares protisches und/oder ein apolares aprotisches und/oder ein polares aprotisches einzusetzen.

Es ist bevorzugt, für die Lösung des 8-{4'-[4''-(Pyrimidin-2'''-yl)-piperazin-1''-yl]-but-2'-inyl}-8-aza-spiro[4,5]decan-7,9-diones der Formel II als Lösungsmittel Methanol und/oder Äthanol (polare[s] protische[s] Lösungsmittel), Benzol (apolares aprotisches Lösungsmittel) und/oder Tetrahydrofuran (polares aprotisches Lösungsmittel) zu verwenden.

Vorzugsweise wird auch für die Suspension des Palladium- oder Raney-Nickel-Katalysators als inertes Lösungsmittel Methanol, Äthanol, Benzol und/oder Tetrahydrofuran verwendet.

Es ist auch bevorzugt, als Palladium-Katalysator einen solchen, welcher auf einen Träger, insbesondere Kohle, aufgebracht ist, zu verwenden.

Besonders bevorzugt wird für die Lösung von 8-{4'-[4''-(Pyrimidin-2'''-yl)-piperazln-1''-yl]-but-2'-inyl}-8-aza-spiro[4,5]decan-7,9-dion der Formel II dasselbe Lösungsmittel verwendet wie für die Suspension des Katalysators.

Nach einer besonders bevorzugten Ausführungsform des erfindungsgemäßen Verfahrens wird die Lösung von 8-{4'-[4''-(Pyrimidin-2'''-yl)-piperazin-1''-yl]-but-2'-inyl}-8-aza-spiro[4,5]decan-7,9-dion der Formel II zur Suspension des Katalysators kontinuierlich mit einer solchen Geschwindigkeit zugegeben, daß das Gewichtsverhältnis des 8-{4'-[4''-(Pyrimidin-2'''-yl)-piperazin-1''-yl]-but-2'-inyl}-8-aza-spiro[4,5]decan-7,9-diones der Formel II zum Katalysator [0,01 bis 1] : 1 beträgt.

Die Hydrierreaktion wird zweckmäßig bei Temperaturen von 10 bis 50°C, vorzugsweise 20 bis 30°C, durchgeführt. Zweckmäßig wird beim Hydrieren ein Druck von 1 bis 10 bar, vorzugsweise 1 bis 5 bar, angewandt.

Das Reaktionsgemisch kann nach verschiedenen Verfahrensweisen aufgearbeitet werden.

Nach einer Ausführungsform des erfindungsgemäßen Verfahrens wird die 8-{4'-[4''-(Pyrimidin-2'''-yl)-piperazin-1''-yl]-butyl}-8-aza-spiro[4,5]decan-7,9-dion-Base der Formel I isoliert [Variante a)]. Dies kann durch Entfernen des Katalysators durch Filtrieren oder Zentrifugieren und darauffolgendes Einedampfen des Filtrates erfolgen. Es kann ohne Um- beziehungsweise Nachkristallisieren eine hochreine 8-{4'-[4''-(Pyrimidin-2'''-yl)-piperazin-1''-yl]-butyl}-8-aza-spiro[4,5]-decan-7,9-dion-Base, welche höchstens 0,1 Gew.-% Verunreinigungen enthält, erhalten werden.

Nach einer anderen Ausführungsform des erfindungsgemäßen Verfahrens wird die 8-{4'-[4''-(Pyrimidin-2'''-yl)-piperazin-1''-yl]-butyl}-8-aza-spiro[4,5]decan-7,9-dion-Base der Formel I bei einer Temperatur von 15 bis 40°C in Äthanol oder Isopropanol mit Chlorwasserstoff behandelt und das bei 188 bis 191°C schmelzende 8-{4'-[4''-(Pyrimidin-2'''-yl)-piperazin-1''-yl]-butyl}-8-aza-spiro[4,5]decan-7,9 dion-hydrochlorid isoliert [Variante b)]. Vorzugsweise wird das Behandeln der 8-{4'-[4''-(Pyrimidin-2'''-yl)-piperazin-1''-yl]-butyl}-8-aza-spiro[4,5]decan-7,9-dion-Base der Formel I in Äthanol oder Isopropanol mit Chlorwasserstoff zur Herstellung des 8-{4'-[4''-(Pyrimidin-2'''-yl)-piperazin-1''-yl]-butyl}-8-aza-spiro[4,5]decan-7,9-dion-hydrochlorides mit einem Schmelzpunkt 188 bis 191°C bei einer Temperatur von etwa 20°C durchgeführt.

Nach einer weiteren Ausführungsform des erfindungsgemäßen Verfahrens wird die 8-{4'-[4''-(Pyrimidin-2'''-yl)-piperazin-1''-yl]-butyl}-8-aza-spiro[4,5]decan-7,9-dion--Base der Formel I bei einer Temperatur von höchstens 70°C in Äthylacetat oder Isopropanol mit Chlorwasserstoff behandelt und das bei 201 bis 203°C schmelzende 8-{4'-[4''-(Pyrimidin-2'''-yl)-piperazin-1''-yl]-butyl}-8-aza-spiro[4,5]decan-7,9-dion-hydrochlorid isoliert [Variante c)]. Vorzugsweise wird das Behandeln der 8-{4'-[4''-(Pyrimidin-2'''-yl)-piperazin-1''-yl]-butyl}-8-aza-spiro[4,5]decan-7,9-dion-Base der Formel I in Äthylacetat oder Isopropanol mit Chlorwasserstoff zur Herstellung des 8-{4'-[4''-(Pyrimidin-2'''-yl)-piperazin-1''-yl]-butyl}-8-aza-spiro[4,5]decan-7,9-dion-hydrochlorides mit einem Schmelzpunkt von 201 bis 203°C bei einer Temperatur von 40 bis 65°C, insbesondere etwa 60°C, durchgeführt.

Es ist auch bevorzugt, die 8-{4'-[4''-(Pyrimidin-2'''-yl)-piperazin-1''-yl]-butyl}-8-aza-spiro[4,5]decan-7,9-dion Base der Formel I zum Überführen in das 8-{4'-[4''-(Pyrimidin-2'''-yl)-piperazin-1''-yl]-butyl}-8-azaspiro[4,5]decan-7,9-dion-hydrochlorid in Form der die 8-{4'-[4''-(Pyrimidin-2'''-yl)-piperazin-1''-yl]-butyl}-8-aza-spiro[4,5]decan-7,9-dion-Base der Formel I enthaltenden, nach dem Entfernen des Katalysators erhaltenen Lösung einzusetzen.

Vorteilhaft wird die Zugabe des Chlorwasserstoff enthaltenden Äthanoles beziehungsweise Isopropanoles beziehungsweise Äthylacetates zur 8-{4'-[4''-(Pyrimidin-2'''-yl)-piperazin-1''-yl]-butyl}-8-aza-spiro[4,5]decan-7,9-dion-Base der Formel I zum Überführen in das 8-{4'-[4''-(Pyrimidin-2'''-yl)-piperazin-1''-yl]-butyl}-8-aza-spiro[4,5]decan-7,9-dion-hydrochlorid 5 bis 30 Minuten lang durchgeführt.

Durch das Rühren wird das Auskristallisieren des 8-{4'-[4''-(Pyrimidin-2'''-yl)piperazin-1''-yl]-butyl}-8-aza-spiro[4,5]decan-7,9-dion-hydrochlorides gefördert.

Es ist auch vorteilhaft, das Rühren beim Behandeln der 8-{4'-[4''-(Pyrimidin-2'''-yl)-piperazin-1''-yl]-butyl}-8-aza-spiro[4,5]decan-7,9-dion-Base der Formel I in Äthanol beziehungsweise Isopropanol beziehungsweise Äthylacetat mit Chlorwasserstoff zur Herstellung des 8-{4'-[4''-(Pyrimidin-2'''-yl)-piperazin-1''-yl]-butyl}-8-aza-spiro[4,5]decan-7,9-dion-hydrochlorides 1 bis 5 Stunden lang durchzuführen.

Die Vorteile des erfindungsgemäßen Verfahrens sind zusammengefaßt insbesondere die folgenden:

a) Größere Ausbeute als nach den bekannten verfahren.

b) Es wird eine hochreine 8-{4'-[4"-(Pyrimidin-2'"-yl)-piperazin-1"-yl]-Butyl}-8-aza-spiro[4,5]decan-7,9-dion-Base erhalten, welche Verunreinigungen nur in minimalen Mengen enthalt.

c) Die erhaltene 8-{4'-[4"-(Pyrimidin-2'"-yl)-piperazin-1"-yl[butyl}-8-aza-spiro[4,5]decan-7,9-dion-Base kann zur Herstellung von polymorphen 8-{4'-[4"-(Pyrimidin-2'"-yl)-piperazin-1"-yl]-butyl}-8-aza-spiro[4,5]decan-7,9-dion-hydrochloriden verschiedener Kristallstruktur unmittelbar, ohne weitere Reinigung eingesetzt werden.

Das erfindungsgemäße Verfahren wird an Hand der folgenden Beispiele näher erläutert, wobei die Angabe F. den in klassischer Weise in einem Kapillarrohr bestimmten Schmelzpunktswertbedeutet und die Bestimmung der 8-{4'-[4"-(Pyrimidin-2'"-yl)-piperazin-1"-yl]-butyl}-8-aza-spiro[4,5]decan-7,9-dion-Base durch Titrieren in wasserfreiem Medium mit Perchlorsäure durchgeführt wurde.

<u>Beispiel 1</u>

Herstellung von 8-{4'-[4"-(Pyrimidin-2'"-yl)-piperazin-1"-yl]-butyl}-8-aza-spiro[4,5]decan-7,9-dion

Es wurde einer Suspension von 4 g eines Palladium/Kohle-Katalysators (Palladiumgehalt: etwa 5 Gew.-%) in 250 ml Methanol eine Lösung von 100 g (0,26 Mol) 8-{4'-[4"-(Pyrimidin-2'"-yl)-piperazin-1"-yl]-but-2'-inyl}-8-aza-spiro [4,5]decan-7,9-dion in 140 ml Methanol unter kräftigem Rühren und unter Wasserstoffzufuhr unter einem Druck von 1 bar mit einer solchen Geschwindigkeit zugesetzt, daß die Wasserstoffaufnahme der zugesetzten ungesättigten Verbindung 8-{4'-[4"-(Pyrimidin-2'"-yl)-piperazin-1"-yl]-but-2'-inyl}-8-aza-spiro[4,5]decan-7,9-dion mit dem berechneten Wasserstoffverbrauch nahezu identisch war (dies wurde mit Hilfe einer Gasbürette kontinuierlich gemessen). Das bedeutet in der Praxis, daß beim Einstellen der Zugabe der Wasserstoffverbrauch binnen 5 bis 10 Sekunden zum Stillstand kommt. Die Zugabe dauerte 2 bis 4 Stunden in Abhängigkeit vom Rühren und der Temperatur (20 bis 30°C).

Nach der Reduktion wurde der Katalysator abfiltriert. Er konnte bei den nächsten Hydrierchargen ohne irgendwelche Behandlung unmittelbar eingesetzt werden. Das Filtrat wurde eingedampft und der Rückstand in Isopropanol suspendiert und abfiltriert.

So wurden 100,3 g 8-{4'-[4"-(Pyrimidin-2'"-yl)-piperazin-1"-yl]-butyl}-8-aza-spiro[4,5]decan-7,9-dion erhalten. Die 8-{4'-[4"-(Pyrimidin-2'"-yl)-piperazin-1"-yl]-butyl}-8-aza-spiro[4,5]decan-7,9-dion-Base ist eine weiße kristalline Substanz.
Ausbeute: etwa 100%.
F.: 105 bis 106°C (nach dem Schrifttum: 90 bis 98°C).
Gehalt (an der Base): 99,8 bis 100,1%.
Auf Grund einer HPLC-Analyse enthielt das Produkt nur etwa 0,1 Gew.-% Verunreinigungen.

<u>Beispiel 2</u>

Herstellung von 8-{4'-[4"-(Pyrimidin-2'"-yl)-piperazin-1"-yl]-butyl}-8-aza-spiro[4,5]decan-7,9-dion-hydrochloriden

Es wurde einer Suspension von 0,5 g eines Palladium/Kohle-Katalysators (Palladiumgehalt: etwa 5 Gew.-%) in 300 ml Äthanol unter kräftigem Rühren und unter Wasserstoffzufuhr unter einem Druck von 5 bar während 1 Stunde eine Lösung von 100 g (0,26 Mol) 8-{4'-[4"-(Pyrimidin-2'"-yl)-piperazin-1"-yl]-but-2'-inyl}-8-aza-spiro[4,5]decan-7,9-dion in 140 ml Äthanol bei Raumtemperatur zugesetzt. Beim Einstellen der Zugabe kam der Wasserstoffverbrauch sofort zum Stillstand. Der Katalysator wurde bei 60°C abfiltriert. Er konnte bei den nächsten Hydrierchangen ohne irgendwelche Behandlung unmittelbar eingesetzt werden.

a) Der erhaltenen Lösung wurden bei 20°C, zu deren Halten erforderlichenfalls ein Kühlen vorgenommen wurde, 50 ml Äthanol mit einem Gehalt an 9,5 g (0,26 Mol) (Chlorwasserstoff während 5 bis 10 Minuten zugesetzt. Nach $1\frac{1}{2}$ Stunden langem Rühren wurde das ausgeschiedene Produkt abfiltriert und getrocknet.
So wurden 107,8 g 8-{4'-[4"-(Pyrimidin-2'"-yl)-piperazin-1"-yl]-butyl}-8-aza-spiro[4,5]decan-7,9-dion-hydrochlorid in Form von weißen Kristallen erhalten.
Ausbeute 97,5%.
F.: 188 bis 189°C.
DSC/10°C/Min/: 190°C.
Gehalt (ermittelt an Hand der Bestimmung des Gehaltes an der Base): 99,9%.
Nach der HPLC-Analyse enthielt das Produkt weniger als 0,1 Gew.-% Verunreinigungen.

Im IR-Spektrum des Produktes war bei 1 150 $cm^{-1}$ keine für das bei 201 bis 203°C schmelzende Polymorph charakteristische Spitze vorhanden.

b) Nach Abfiltrieren des Katalysators von der im ersten Absatz erhaltenen Lösung der 8-{4'-[4"-(Pyrimidin-2'''-yl)-piperazin-1"-yl]-butyl}-8-aza-spiro[4,5]decan-7,9-dion-Base wurde das Lösungsmittel gegen 1 000 ml Isopropanol ausgetauscht, worauf der Suspension bei 20°C, zu deren Halten erforderlichenfalls ein Kühlen vorgenommen wurde, 50 ml Isopropanol mit einem Gehalt an 9,5 g (0,26 Mol) Chlorwasserstoff während 15 Minuten zugesetzt wurde.
Nach $1\frac{1}{2}$-stündigem Weiterrühren wurden durch Filtrieren und Trocknen des Filterrückstandes 106,2 g 8-{4'-[4"-(Pyrimidin-2'''-yl)-piperazin-1"-yl]-butyl}-8-aza-spiro[4,5]decan-7,9-dion-hydrochlorid in Form von weißen Kristallen isoliert.
Ausbeute: 96,8%.
Das Produkt war mit der unter Verwendung von Äthanol erhaltenen Substanz (Schmelzpunkt, Reinheit) identisch.

c) Nach Abfiltrieren des Katalysators von der im ersten Absatz erhaltenen Lösung der 8-{4'[4"-(Pyrimidin-2'''-yl)-piperazin-1"-yl]-butyl}-8-aza-spiro[4,5]decan-7,9-dion-Base wurde das Lösungsmittel gegen 500 ml Äthylacetat ausgetauscht, worauf der Suspension bei 60°C Äthylacetat mit einem Gehalt an 9,49 g (0,26 Mol) Chlorwasserstoff während 10 Minuten zugesetzt wurde.
Nach $1\frac{1}{2}$-stündigem Rühren wurden aus der Lösung durch Filtrieren und Trocknen des Filterrückstandes 94 g 8-{4'-[4"-(Pyrimidin-2'''-yl)-piperazin-1"-yl]-butyl}-8-aza-spiro[4,5]decan-7,9-dion-hydrochlorid in Form von weißen Kristallen isoliert.
Ausbeute 85%.
F.: 203 bis 204°C.
DSC/10°C/Min./: 206,59°.
Nach der HPLC-Analyse enthielt das Produkt weniger als 0,1 Gew.-% Verunreinigungen.
Gehalt (ermittelt an Hand der Bestimmung des Gehaltes an der Base): 99,5 bis 100,5%.
Im IR-Spektrum des Produktes war bei 1 290 $cm^{-1}$ keine für das bei 188 bis 191°C schmelzende Polymorph charakteristische Spitze vorhanden.

d) Es wurde wie im Abschnitt c) beschrieben vorgegangen, jedoch mit dem Unterschied, daß an Stelle des Äthylacetates Isopropanol verwendet wurde.
So wurden 86,4 g 8-{4'-[4"-(pyrimidin-2'''-yl)-piperazin-1"-yl]-butyl}-8-aza-spiro[4,5]decan-7,9-dion-hydrochlorid erhalten.
F.: 203 bis 204°C.
Die Reinheit des Produktes war mit derselben des nach Abschnitt c) hergestellten 8-{4'-[4"-(Pyrimidin-2'''-yl)-piperazin-1"-yl]-butyl}-8-aza-spiro[4,5]decan-7,9-dion-hydrochlorides identisch.

Beispiel 3

Herstellung von 8-{4'-[4"-(Pyrimidin-2'''-yl)piperazin-1"-yl]-butyl}-8-aza-spiro[4,5]decan-7,9-dion

Es wurde einer Suspension von 8 g Raney-Nickel in 250 ml Methanol unter kräftigem Rühren und unter Wasserstoffzufuhr unter einem Druck von 5 bar eine Lösung von 100 g (0,26 Mol) 8-{4'-[4"-(Pyrimidin-2'''-yl)-piperazin-1"-yl]-but-2'-inyl}-8-aza-spiro[4,5]decan-7,9-dion in 140 ml Methanol bei 20 bis 40°C mit einer solchen Geschwindigkeit zugesetzt, daß die Wasserstoffaufnahme der ungesättigten Verbindung 8-{4'-[4"-(Pyrimidin-2'''-yl)-piperazin-1"-yl]-but-2'-inyl} -8-aza-spiro[4,5]decan-7,9-dion mit dem kontinuierlich gemessenen berechneten Wasserstoffverbrauch nahezu identisch war. Die Zugabe dauerte 1 bis 2 Stunden. Dies bedeutet in der Praxis, daß bei Einstellen der Zugabe die Erniedrigung des Druckes im Apparat innerhalb einiger Sekunden zum Stillstand kam. Die Zeitdauer der Zugabe betrug 1 bis 2 Stunde(n) in Abhängigkeit von der Intensität des Rührens. Die Reaktionstemperatur lag zwischen 20 und 40°C.
Am Ende der Reduktion wurde der Katalysator abfiltriert. Er konnte bei den nächsten Hydrierchangen ohne irgendwelche Behandlung unmittelbar eingesetzt werden. Das Filtrat wurde eingedampft und der Rückstand in Isopropanol suspendiert und abfiltriert.
So wurden 93,7 g 8-{4'-[4"-(Pyrimidin-2'''-yl)-piperazin-1"-yl]-butyl}-8-aza-spiro[4,5]decan-7,9-dion-Base in Form von weißen Kristallen erhalten.
Ausbeute 98,9%.
F.: 105 bis 106°C.
Gehalt (an der Base): 99,9%.
Auf Grund der HPLC-Analyse enthielt das Produkt nur 0,1 Gew.-% Verunreinigungen.

Beispiel 4

Herstellung von 8-{4'-[4"-(Pyrimidin-2'"-yl)-piperazin-1"-yl]-butyl}-8-aza-spiro[4,5]decan-7,9-dion

Es wurde einer Suspension von 2 g eines Palladium/Kohle-Katalysators (Palladiumgehalt etwa 5 Gew.-%) in 50 ml thiophenfreiem Benzol unter kräftigem Rühren bei 20 bis 40°C unter Wasserstoffzufuhr unter einem Druck von 1 bar eine Lösung von 100,0 g (0,26 Mol) 8-{4'-[4"-(Pyrimidin-2'"-yl)piperazin-1"-yl]-but-2'-inyl}-8-aza-spiro[4,5]decan-7,9-dion in 50 ml thiophenfreiem Benzol mit einer solchen Geschwindigkeit zugesetzt, daß die Wasserstoffaufnahme der zugesetzten ungesättigten Verbindung 8-{4'-[4"-(Pyrimidin-2'"-yl)-piperazin-1"-yl]-but-2'-inyl}-8-aza-spiro[4,5]decan-7,9-dion mit dem berechneten Wasserstoffverbrauch, welcher mit Hilfe einer Gasbürette kontinuierlich gemessen wurde, nahezu identisch war. Dies bedeutet in der Praxis, daß nach Einstellen (Abbrechen) der Zugabe der Wasserstoffverbrauch während 5 bis 10 Sekunden zum Stillstand kam. Die Reaktionstemperatur lag zwischen 20 und 40°C und die Reaktionszeit betrug 1 bis 4 Stunde(n). Am Ende der Reduktion wurde der Katalysator abfiltriert. Er konnte bei den nächsten Hydrierchargen ohne irgendwelche Behandlung unmittelbar eingesetzt werden. Das Filtrat wurde eingedampft und der Rückstand in Isopropanol suspendiert und abfiltriert.

So wurden 100,3 g 8-{4'-[4"-(Pyrimidin-2'"-yl)-piperazin-1"-yl]-butyl}-8-aza-spiro[4,5]decan-7,9-dion-Base in Form von weißen Kristallen erhalten.
Ausbeute: etwa 100%.
F.: 105 bis 106°C (nach dem Schrifttum 90 bis 98°C).
Gehalt (an der Base): 99,8 bis 100,1%.
Auf Grund der HPLC-Analyse enthielt das Produkt nur 0,1% Verunreinigungen.

Beispiel 5

Herstellung von 8-{4'-[4"-(Pyrimidin-2'"-yl)-piperazin-1"-yl]-butyl}-8-aza-spiro[4,5]decan-7,9-dion

Es wurde einer Suspension von 1 g eines Palladium/Kohle-Katalysators (Palladiumgehalt: etwa 5 Gew.-%) in 50 ml Tetrahydrofuran unter kräftigem Rühren und unter Wasserstoffzufuhr unter einem Druck von 2 bar eine Lösung von 100 g (0,26 Mol) 8-{4'-[4"-(Pyrimidin-2'"-yl)-piperazin-1"-yl]-but-2'-inyl}-8-aza-spiro[4,5]decan-7,9-dion in 50 ml Tetrahydrofuran mit einer solchen Geschwindigkeit zugesetzt, daß die Wasserstoffaufnahme der zugesetzten ungesättigten Verbindung 8-{4'-[4"-(Pyrimidin-2'"-yl)-piperazin-1"-yl]-but-2'-inyl}-8-aza-spiro[4,5]decan-7,9-dion mit dem berechneten Wasserstoffverbrauch nahezu identisch war. Dies bedeutet in der Praxis, daß bei Einstellen der Zugabe die Veiminderung des Wasserstoffdruckes im Apparat innerhalb 5 bis 10 Sekunden zum Stillstand kam. Die Zugabe dauerte 1 bis 4 Stunde(n) in Abhängigkeit von der Intensität des Rührens. Die Reaktionstemperatur wurde während der ganzen Dauer der Zugabe zwischen 15 und 30°C gehalten. Am Ende der Reduktion wurde der Katalysator abfiltriert. Er konnte bei den nächsten Hydrierchargen ohne irgendwelche Behandlung unmittelbar eingesetzt werden. Das Filtrat wurde eingedampft und der Rückstand in Isopropanol suspendiert und abfiltriert.

So wurden 99,7 g 8-{4'-[4"-(Pyrimidin-2'"-yl)-piperazin-1"-yl]-butyl}-8-aza-spiro[4,5]decan-7,9-dion in Form von weißen Kristallen erhalten.
Ausbeute: 98,7%
F.: 105 bis 106°C (nach dem Schrifttum: 90 bis 98°C).
Gehalt (an der Base): 99,8 bis 100,1%.
Auf Grund der HPLC-Analyse enthielt das Produkt nur 0,1% Verunreinigungen.

Vergleichsbeispiel

Herstellung von 8-{4'-[4"-(Pyrimidin-2'"-yl)-piperazin-1"-yl]-butyl}-8-aza-spiro[4,5]decan-7,9-dion (nach DOS 3 806 009)

Es wurde einer Lösung von 38,15 g (0,1 Mol) 8-{4'-[4"-(Pyrimidin-2'"-yl)-piperazin-1"-yl]-but-2'-inyl}-8-aza-spiro[4,5]decan-7,9-dion in 150 ml Äthanol 1 g eines Palladium/Kohle-Katalysators zugesetzt. Die Suspension wurde bei Raumtemperatur und unter Atmosphärendruck unter kräftigem Rühren so lange hydriert, bis der Wasserstoffverbrauch aufhörte (2 Äquivalente Wasserstoff, etwa 5,0 l). Der Katalysator wurde abfiltriert und konnte bei den nächsten Hydrierstufen ohne irgendwelche Behandlung unmittelbar eingesetzt werden. Das Lösungsmittel wurde unter vermindertem Druck abgedampft.

So wurden 36,85 g 8-{4'-[4"-(Pyrimidin-2'"-yl)-piperazin-1"-yl]-butyl}-8-aza-spiro[4,5]decan-7,9-dion erhalten.
Ausbeute: 95,6%.
F.: 91 bis 99°C (nach dem Schrifttum 90 bis 98°C).

Gehalt (an der Base): 102,86%.
Auf Grund der HPLC-Analyse enthielt das Produkt die folgenden Verbindungen:
98,33 Gew.-% des gewünschten Produktes 8-{4'-[4"-(Pyrimidin-2'''-yl)-piperazin-1"-yl]-butyl}-8-aza-spiro[4,5]decan-7,9-dion.
0,85 Gew.-% 8-{4'-[4"-(Pyrimidin-2'''-yl)-piperazin-1"-yl]-but-2'-enyl}-8-aza-spiro[4,5]decan-7,9-dion.
0,42 Gew.-% 8-{4'-[4"-(3''',4''',5''',6'''-Tetra-⟨hydro⟩-pyrimidin-2'''-yl)-piperazin-1"-yl]-butyl}-8-aza-spiro[4,5]decan-7,9-dion.
0,40 Gew.-% weitere Substanzen unbekannter Struktur.

Ein Produkt pharmazeutischer Reinheit konnte nur durch 2-maliges Umkristallisieren aus Isopropanol hergestellt werden.
Ausbeute: 25,9 g (67,2%).
F.: 105 bis 106°C.
Gehalt (an der Base): 99,8 bis 100,0%.
Nach der HPLC-Analyse enthielt das Produkt nun 0,1% Verunreinigungen.

## Patentansprüche

1. Verfahren zur Herstellung von 8-{4'-[4"-(Pyrimidin--2'''-yl)-piperazin-1"-yl]-butyl}-8-aza-spiro[4,5]decan--7,9-dion {Buspiron} der Formel

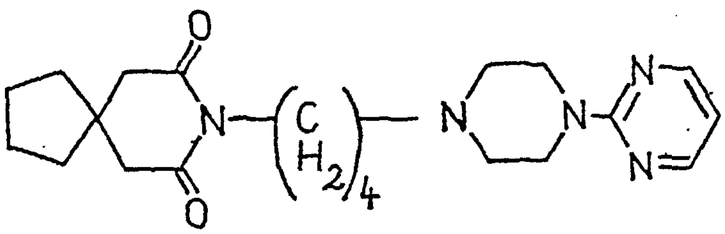

I

und von dessen Hydrochloriden hoher Reinheit durch katalytisches Hydrieren von 8-{4'-[4"-(Pyrimidin-2'''--yl)-piperazin-1"-yl]-but-2'-inyl}-8-aza--spiro[4,5]decan-7,9-dion der Formel

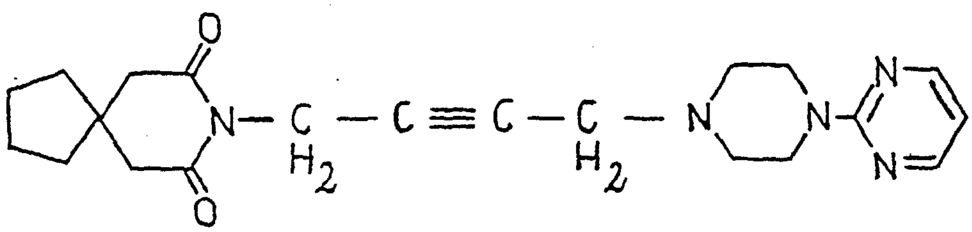

II

in Gegenwart eines Palladium- oder Raney-Nickel-Katalysators in einem inerten organischen Lösungsmittel und gegebenenfalls darauffolgendes Überführen des erhaltenen 8-{4'-[4"-(Pyrimidin-2'''-yl)-piperazin--1"-yl]-butyl}-8-aza-spiro[4,5)decan-7,9-diones in ein Hydrochlorid, dadurch gekennzeichnet, daß man eine Lösung von 8-{4'-[4"-(Pyrimidin-2'''-yl)-piperazin--1"-yl]-but-2'-inyl}-8-aza-spiro[4,5]decan-7,9-dion der Formel II in einem inerten organischen Lösungsmittel, deren Konzentration mindestens 40 Gew./Gew.-% ist, einer Suspension des Katalysators in einem inerten organischen Lösungsmittel kontinuierlich zusetzt, den Katalysator entfernt und danach

a) die 8-{4'-[4"-(Pyrimidin-2'''-yl)-piperazin-1"-yl]-butyl}-8-aza-spiro[4,5]decan-7,9-dion-Base der Formel I isoliert und/oder

b) die 8-{4'-[4"-(Pyrimidin-2'''-yl)-piperazin-1"-yl]-butyl}-8-aza-spiro[4,5]decan-7,9-dion-Base der Formel I bei einer Temperatur von 15 bis 40°C in Äthanol oder Isopropanol mit Chlorwasserstoff unter Rühren behandelt und das bei 188 bis 191°C schmelzende 8-{4'-4"-(Pyrimidin-2'''-yl)-piperazin-1"-yl]-butyl}-8-aza-spiro[4,5]decan-7,9-dion-hydrochlorid isoliert oder

c) die 8-{4'-[4"-(Pyrimidin-2'''-yl)-piperazin-1"-yl]-butyl}-8-aza-spiro[4,5] decan-7,9-dion-Base der Formel I bei einer Temperatur von höchstens 70°C in Äthylacetat oder Isopropanol mit Chlorwasserstoff unter Rühren behandelt und das bei 201 bis 203°C schmelzende 8-{4'-[4"-(Pyrimidin-2'''-yl)-piperazin-1"-yl]-butyl}-8-aza-spiro[4,5]decan-7,9-dion-hydrochlorid isoliert.

**2.** Verfahren nach Anspruch 1, dadurch gekennzeichnet, daß man eine Lösung mit einer Konzentration von 40 bis 70 Gew./Gew.-% am 8-{4'-[4"-(Pyrimidin-2'''-yl)-piperazin-1"-yl]-but-2'-inyl}-8-aza-spiro[4,5]decan-7,9-dion der Formel II verwendet.

**3.** Verfahren nach Anspruch 1 oder 2, dadurch gekennzeichnet, daß man für die Lösung des 8-{4'-[4"-(Pyrimidin-2'''-yl)-piperazin-1"-yl]-but-2'-inyl}-8-aza -spiro[4,5]decan-7,9-diones der Formel II als Lösungsmittel ein polares protisches und/oder ein apolares aprotisches und/oder ein polares aprotisches einsetzt.

**4.** Verfahren nach Anspruch 1 bis 3, dadurch gekennzeichnet, daß man für die Lösung des 8-{4'-[4"-(Pyrimidin-2'''-yl)-piperazin-1"-yl]-but-2'-inyl}-8-aza-spiro[4,5]decan-7,9-diones der Formel II als Lösungsmittel Methanol, Äthanol, Benzol und/oder Tetrahydrofuran einsetzt.

**5.** Verfahren nach Anspruch 1 bis 4, dadurch gekennzeichnet, daß man für die Suspension des Palladium- oder Raney-Nickel-Katalysators als inertes Lösungsmittel Methanol, Äthanol, Benzol und/oder Tetrahydrofuran verwendet.

**6.** Verfahren nach Anspruch 1 bis 5, dadurch gekennzeichnet, daß man als Palladium-Katalysator einen solchen, welcher auf einen Träger, insbesondere Kohle, aufgebracht ist, verwendet.

**7.** Verfahren nach Anspruch 1 bis 6, dadurch gekennzeichnet, daß man für die Lösung von 8-{4'-[4"-(Pyrimidin-2'''-yl)-piperazin-1"-yl]-but-2'-inyl}-8-aza-spiro[4,5]decan-7,9-dion der Formel II dasselbe Lösungsmittel verwendet wie für die Suspension des Katalysators.

**8.** Verfahren nach Anspruch 1 bis 7, dadurch gekennzeichnet, daß man die Lösung von 8-{4'-[4"-(Pyrimidin-2'''-yl)-piperazin-1"-yl]-but-2'-inyl}-8-aza-spiro[4,5]decan-7,9-dion der Formel II zur Suspension des Katalysators kontinuierlich mit einer solchen Geschwindigkeit zugibt, daß das Gewichtsverhältnis des 8-{4'-[4"-(Pyrimidin-2'''-yl)-piperazin-1"-yl]-but-2'-inyl}-8-aza-spiro[4,5]decan-7,9-diones der Formel II zum Katalysator /0.01 bis 1/ : 1 beträgt.

**9.** Verfahren nach Anspruch 1b) oder 2 bis 8, dadurch gekennzeichnet, daß man das Behandeln der 8-{4'-[4"-(Pyrimidin-2'''-yl)-piperazin-1"-yl]-butyl}-8-aza-spiro[4,5]decan-7,9-dion-Base der Formel I in Äthanol oder Isopropanol mit Chlorwasserstoff zur Herstellung des 8-{4'-[4"-(Pyrimidin-2'''-yl)-piperazin-1"-yl]-butyl}-8-aza-spiro[4,5]decan-7,9-dion-hydrochlorides mit einem Schmelzpunkt von 188 bis 191°C bei einer Temperatur von etwa 20°C durchführt.

**10.** Verfahren nach Anspruch 1c)oder 2 bis 8, dadurch gekennzeichnet, daß man das Behandeln der 8-{4'-[4"-(Pyrimidin-2'''-yl)-piperazin-1"-yl]-butyl}-8-aza-spiro[4,5]decan-7,9-dion-Base der Formel I in Äthylacetat oder Isopropanol mit Chlorwasserstoff zur Herstellung des 8-{4'-[4"-(Pyrimidin-2'''-yl)-piperazin-1"-yl]-butyl}-8-aza-spiro[4,5] decan-7,9-dion-hydrochlorides mit einem Schmelzpunkt von 201 bis 203°C bei einer Temperatur von 40 bis 65°C, insbesondere etwa 60°C, durchführt.

**11.** Verfahren nach Anspruch 1b) oder 1c) oder 2 bis 10, dadurch gekennzeichnet, daß man die 8-{4'-[4"-(Pyrimidin-2'''-yl)-piperazin-1"-yl]-butyl}-8-aza-spiro[4,5]decan-7,9-dion-Base der Formel I zum Überführen in das 8-{4'-[4"-(Pyrimidin-2'''-yl)-piperazin-1"-yl]-butyl}-8-aza-spiro[4,5]decan-7,9-dion-hydrochlorid in Form der die 8-{4'-[4"-(Pyrimidin-2'''-yl)-piperazin-1"-yl]-butyl}-8-aza-spiro[4,5]decan-7,9--dion-Base der Formel I enthaltenden, nach dem Entfernen des Katalysators erhaltenen Lösung einsetzt.

**12.** Verfahren nach Anspruch 1b) oder 1c) oder 2 bis 11, dadurch gekennzeichnet, daß man die Zugabe des Chlorwasserstoff enthaltenden Äthanoles beziehungsweise Isopropanoles beziehungsweise Äthylacetates zur 8-{4'-[4"-(Pyrimidin-2'''-yl)-piperazin-1"-yl]-butyl}-8-aza-spiro[4,5]decan-7,9-dion-Base der Formel I zum Überführen in das 8-{4'-[4"-(Pyrimidin-2'''-yl)-piperazin-1"-yl]-butyl}-8-aza-spiro[4,5]decan-7,9-dion-hydrochlorid 5 bis 30 Minuten lang durchführt.

**13.** Verfahren nach Anspruch 1b) oder 1c) oder 2 bis 12, dadurch gekennzeichnet, daß man das Rühren beim Behandeln der 8-{4'-[4"-(Pyrimidin-2'"-yl)-piperazin-1"-yl]-butyl}-8-aza-spiro[4,5]decan-7,9-dion-Base der Formel I in Äthanol beziehungsweise Isopropanol beziehungsweise Äthylacetat mit Chlorwasserstoff zur Herstellung des 8-{4'-[4"-(Pyrimidin-2'"-yl)-piperazin-1"-yl]-butyl}-8-aza-spiro[4,5]decan-7,9-dion-hydrochlorides 1 bis 5 Stunden lang durchführt.

## Claims

1. A process for the production of 8-{4'-[4"-(pyrimidine-2'"-yl)-piperazine-1"-yl]-butyl}-8-aza-spiro[4,5]decane-7,9-dione-{buspirone}having the formula

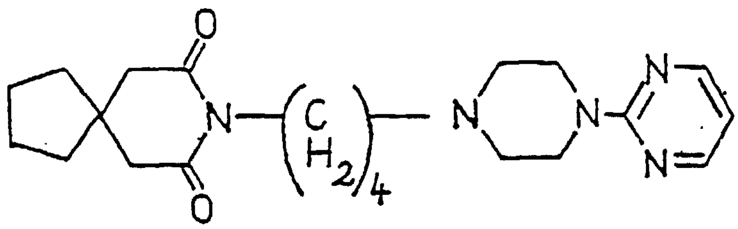

I

and of the high purity hydrochlorides thereof by catalytic hydrogenation of 8-{4'-[4"-(pyrimidine-2'"-yl)-piperazine-1"-yl]-but-2'-inyl}-8-aza-spiro[4,5]decane-7, 9-dione having the formula

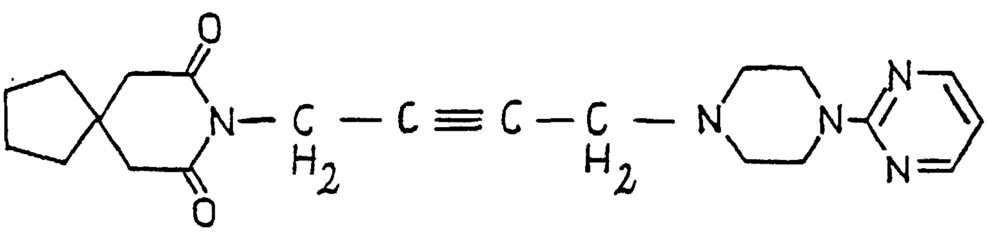

II

in the presence of a palladium or Raney nickel catalyst in an inert organic solvent and optionally subsequently converting said 8-{4'-[4"-(pyrimidine-2'"-yl)-piperazine-1"-yl]-butyl}-8-aza-spiro[4,5]decane-7,9-dione obtained into a hydrochloride, characterized by continuously adding a solution of 8-{4'-[4"-(pyrimidine-2'"-yl)-piperazine-1"-yl]-but-2'-inyl}-8-aza-spiro[4,5]decane-7,9-dione of formula II in an inert organic solvent, the concentration of which is at least 40 % by weight/ % by weight, to a suspension of the catalyst in an inert organic solvent, removing said catalyst and thereafter

a) isolating said 8-{4'-[4"-(pyrimidine-2'"-yl)-piperazine-1"-yl]-butyl}-8-aza-spiro[4,5]decane 7,9-dione base of formula I, and/or

b) treating said 8-{4'-[4"-(pyrimidine-2'"-yl)-piperazine-1"-yl]-butyl}-8-aza-spiro[4,5]decane-7,9-dione base of formula I at a temperature of 15 to 40°C in ethanol or isopropanol with hydrogen chloride with stirring, and isolating the 8-{4'-[4"-(pyrimidine-2'"-yl)-piperazine-1"-yl]-butyl}-8-aza-spiro[4,5]decane-7,9-dione hydrochloride melting from 188 to 191°C, or

c) treating said 8-{4'-[4"-(pyrimidine-2'"-yl)-piperazine-1"-yl]-butyl}-8-aza-spiro[4,5]decane-7,9-dione base of formula I at a temperature of at maximum 70°C in ethyl acetate or isopropanol with hydrogen chloride with stirring, and isolating said 8-{4'-[4"-(pyrimidine-2'"-yl)-piperazine-1"-yl]-butyl}-8-aza-spiro[4,5]decane-7,9-dione hydrochloride melting from 201 to 203°C.

2. A process according to claim 1, characterized in that a solution with a concentration of 40 to 70 % by weight/% by weight of 8-{4'-[4"-(pyrimidine-2'"-yl)-piperazine-1"-yl]-but-2'-inyl}-8-aza-spiro[4,5]decane-7,9-dione of formula II is used.

3. A process according to claim 1 or 2, characterized in that for the solution of said 8-{4'-[4"-(pyrimidine-2'"-yl)-piperazine-1"-yl]-but-2'-inyl}-8-aza-spiro[4,5]decane-7,9-dione of formula II a polar protic and/or an apolar aprotic and/or a polar aprotic one is used as a solvent.

4. A process according to any one of claims 1 to 3, characterized in that for the solution of 8-{4'-[4"(pyrimidine-2'''-yl)-piperazine-1"-yl]-but-2'-inyl}-8-aza-spiro[4,5]decane-7,9-dione of formula II methanol, ethanol, benzene, and/or tetrahydrofuran is used as a solvent.

5. A process according to any one of claims 1 to 4, characterized in that for the suspension of palladium or Raney nickel catalyst methanol, ethanol, benzene, and/or tetrahydrofuran is used as an inert solvent.

6. A process according to any one of claims 1 to 5, characterized in that, as a palladium catalyst, there is used one which is applied onto a carrier, especially coal.

7. A process according to any one of claims 1 to 6, characterized in that for the solution of 8-{4'-[4"-(pyrimidine-2'''-yl)-piperazine-1"-yl]-but-2'-inyl}-8-aza-spiro[4,5]decane-7,9-dione of formula II there is used the same solvent as for the suspension of the catalyst.

8. A process according to any one of claims 1 to 7, characterized in that the solution of 8-{4'-[4"-(pyrimidine-2'''-yl)-piperazine-1"-yl]-but-2'-inyl}-8-aza-spiro[4,5]decane-7, 9-dione of formula II is added continuously to the suspension of the catalyst at such a rate that the weight ratio of 8-{4'-[4"-(pyrimidine-2'''-yl)-piperazine-1"-yl]-but-2'-inyl}-8-aza-spiro[4,5]decane-7,9-dione of formula II to the catalyst is /0.01 to 1/ : 1.

9. A process according to any one of claims 1b) or 2 to 8, characterized in that the treatment of said 8-{4'-[4"-(pyrimidine-2'''-yl)-piperazine-1"-yl]-butyl}-8-aza-spiro[4,5]decane-7,9-dione base of formula I is carried out in ethanol or isopropanol with hydrogen chloride for the production of said 8-{4'-[4"-(pyrimidine-2'''-yl)-piperazine-1"-yl]-butyl}-8-aza-spiro[4,5]decane-7,9-dione hydrochloride having a melting point from 188° to 191°C at a temperature of about 20°C.

10. A process according to any one of claims 1c) or 2 to 8, characterized in that the treatment of said 8-{4'-[4"-(pyrimidine-2'''-yl)-piperazine-1"-yl]-butyl}-8-aza-spiro[4,5]decane-7,9-dione base of formula I is carried out in ethyl acetate or isopropanol with hydrogen chloride for the production of said 8-{4'-[4"-(pyrimidine-2'''-yl)-piperazine-1"-yl]-butyl}-8-aza-spiro[4,5]decane-7,9-dione hydrochloride having a melting point from 201 to 203°C at a temperature of 40 to 65°C, especially at about 60°C.

11. A process according to any one of claims 1b) or 1c) or 2 to 10, characterized in that said 8-{4'-[4"-(pyrimidine-2'''-yl)-piperazine-1"-yl]-butyl}-8-aza-spiro[4,5]decane-7,9-dione base of formula I is used for the conversion into said 8-{4'-[4"-(pyrimidine-2'''-yl)-piperazine-1"-yl]-butyl}-8-aza-spiro[4,5]decane-7,9-dione hydrochloride in the form of said 8-{4'-[4"-(pyrimidine-2'''-yl)-piperazine-1"-yl]-butyl}-8-aza-spiro[4,5]decane-7,9-dione base of formula I containing solution being obtained after removing the catalyst.

12. A process according to any one of claims 1b) or 1c) or 2 to 11, characterized in that the addition of hydrogen chloride-containing ethanol or isopropanol or ethyl acetate, respectively, to 8-{4'-[4"-(pyrimidine-2'''-yl)-piperazine-1"-yl]-butyl}-8-aza-spiro[4,5]decane-7,9-dione base of formula I for the conversion into 8-{4'-[4"-(pyrimidine-2'''-yl)-piperazine-1"-yl]butyl}-8-aza-spiro[4,5]decane-7,9-dione hydrochloride is performed for 5 to 30 minutes.

13. A process according to any one of claims 1b) or 1c) or 2 to 12, characterized in that the stirring while treating the 8-{4'-[4"-(pyrimidine-2'''-yl)-piperazine-1"-yl]-butyl}-8-aza-spiro[4,5]decane-7,9-dione base of formula I in ethanol or isopropanol or ethyl acetate, respectively, with hydrogen chloride for the production of said 8-{4'-[4"-(pyrimidine-2'''-yl)piperazine-1"-yl]-butyl}-8-aza-spiro[4,5]decane-7,9-dione hydrochloride is performed for 1 to 5 hours.

**Revendications**

1. Procédé de préparation de 8-{4'-[4"-(pyrimidine-2'''-yle)-pipérazine-1"-yle]-butyle}-8-aza-spiro[4,5]décane-7,9-dione {Buspiron} de la formule

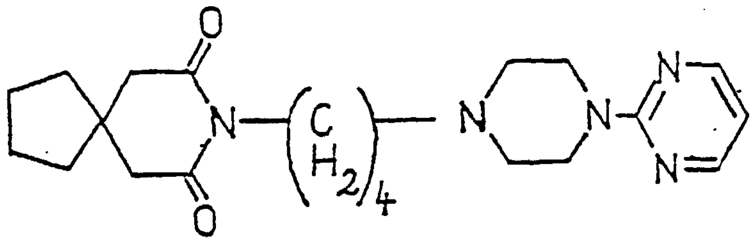

I

et de ses hydrochlorures de haute pureté par l'hydrogénation catalytique de 8-{4'-[4"-(pyrimidine-2'''-yle)-pipérazine-1"-yle]-but-2'-inyle}-8-aza-spiro[4,5]décane-7,9-dione de la formule

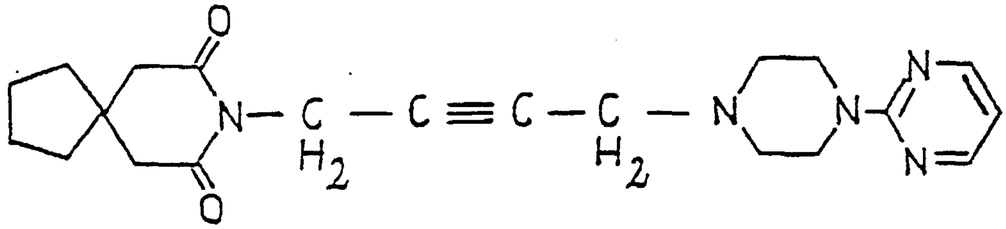

II

en présence d'un catalyseur de palladium ou de Raney nickel dans un solvant organique inerte et le cas échéant la transformation successive du 8-{4'-[4"-(pyrimidine-2'''-yle)-pipérazine-1"-yle]-butyle}-8-aza-spiro[4,5]décane-7,9-dione en un hydrochlorure, caractérisé en ce qu'on ajoute continuellement une solution de 8-{4'-[4"-(pyrimidine-2'''-yle)-pipérazine-1"-yle]-but-2'-inyle}-8-aza-spiro[4,5]décane-7,9-dione de la formule II dans un solvant organique inerte, dont la concentration est au moins de 40 poids/poids-%, à une suspension du catalyseur dans un solvant organique inerte, qu'on élimine le catalyseur et qu'en suite

a) on isole la base de 8-{4'-[4"-(pyrimidine-2'''-yle)-pipérazine-1"-yle]-butyle}-8-aza-spiro[4,5]décane-7,9-dione de la formule I et/ou

b) traite la base de 8-{4'-[4"-(pyrimidine-2'''-yle)-pipérazine-1"-yle]-butyle}-8-aza-spiro[4,5]décane-7,9-dione de la formule I à une température de 15 à 40°C dans de l'éthanol ou de l'isopropanol avec du gaz hydrochlorique en brassant et isole l'hydrochlorure de 8-{4'-[4"-(pyrimidine-2'''-yle)-pipérazine-1"-yle]butyle}-8-aza-spiro[4,5]décane-7,9-dione fondant à une température de 188 à 191°C ou

c) traite la base de 8-{4'-[4"-(pyrimidine-2'''-yle)-pipérazine-1"-yle]-butyle}-8-aza-spiro[4,5]décane-7,9-dione de la formule I à une température maximale de 70°C dans de l'acétate éthylique ou de l'isopropanol avec du gaz hydrochlorique en brassant et isole l'hydrochlorure de 8-{4'-[4"-(pyrimidine-2'''-yle)pipérazine-1"-yle]-butyle}-8-aza-spiro[4,5]décane-7,9-dione fondant à une température de 201 à 203°C.

**2.** Procédé selon la revendication 1, caractérisé en ce qu'on utilise une solution présentant une concentration de 40 à 70 poids/poids-% sur le 8-{4'-[4"-(pyrimidine-2'''-yle)-pipérazine-1"-yle]-but-2'-inyle}-8-aza-spiro[4,5]décane-7,9-dione de la formule II.

**3.** Procédé selon les revendications 1 ou 2, caractérisé en ce qu'on utilise pour la solution du 8-{4'-[4"-(pyrimidine-2'''-yle)-pipérazine-1"-yle]-but-2'-inyle}-8-aza-spiro[4,5]décane-7,9-dione de la formule II comme solvant un du type polaire protique et/ou apolaire aprotique et/ou polaire aprotique.

**4.** Procédé selon les revendications 1 à 3, caractérisé en ce qu'on utilise pour la solution du 8-{4'-[4"-(pyrimidine-2'''-yle)-pipérazine-1"-yle]-but-2'-inyle}-8-aza-spiro[4,5] décane-7,9-dione de la formule II comme solvant du méthanol, de l'éthanol, du benzène et/ou du tétrahydrofuranne.

**5.** Procédé selon les revendications 1 à 4, caractérisé en ce qu'on utilise pour la suspension du catalyseur de palladium ou de Raney nickel comme solvant inerte du méthanol, de l'éthanol, du benzène et/ou du tétrahydrofuranne.

6. Procédé selon les revendications 1 à 5, caractérisé en ce qu'on utilise comme catalyseur de palladium un du type qui est appliqué sur un substrat, notamment du charbon.

7. Procédé selon les revendications 1 à 6, caractérisé en ce que pour la solution du 8-{4'-[4"-(pyrimidine-2'''-yle)-pipérazine-1"-yle]-but-2'-inyle}-8-aza-spiro[4,5] décane-7,9-dione de la formule II on utilise le même solvant que pour la suspension du catalyseur.

8. Procédé selon les revendications 1 à 7, caractérisé en ce qu'on ajoute la solution de 8-{4'-[4"-(pyrimidine-2'''-yle)-pipérazine-1"-yle] -but-2'-inyle}-8-aza-spiro[4,5]décane-7,9-dione de la formule II à la suspension du catalyseur de manière continue, à une vitesse telle que le rapport de poids du 8-{4'-[4"-(pyrimidine-2'''-yle)-pipérazine-1"-yle]-but-2'-inyle}-8-aza-spiro[4,5]décane-7,9-dione de la formule II au catalyseur est de /0,01 à 1/ : 1.

9. Procédé selon les revendications 1b) ou 2 à 8, caractérisé en ce qu'on exécute le traitement de la base de 8-{4'-[4"-(pyrimidine-2'''-yle)-pipérazine-1"-yle]-butyle}-8-aza-spiro[4,5]décane-7,9-dione de la formule I dans de l'éthanol ou de l'isopropanol avec du gaz hydrochlorique pour la préparation de l'hydrochlorure de 8-{4'-[4"-(pyrimidine-2'''-yle)-pipérazine-1"-yle]-butyle}-8-aza-spiro[4,5]décane-7,9-dione présentant un point de fusion de 188 à 191°C à une température d'environ 20°C.

10. Procédé selon les revendications 1c) ou 2 à 8, caractérisé en ce qu'on exécute le traitement de la base de 8-{4'-[4"-(pyrimidine-2'''-yle)-pipérazine-1"-yle]-butyle}-8-aza-spiro[4,5]décane-7,9-dione de la formule I dans de l'acétate éthylique ou de l'isopropanol avec du gaz hydrochlorique pour la préparation de l'hydrochlorure de 8-{4'-[4"-(pyrimidine-2'''-yle)-pipérazine-1"-yle]-butyle}-8-aza-spiro[4,5]décane-7,9-dione présentant un point de fusion de 201 à 203°C à une température de 40 à 65°C, notamment à environ 60°C.

11. Procédé selon les revendications 1b) ou 1c) ou 2 à 10, caractérisé en ce qu'on utilise la base de 8-{4'-[4"-(pyrimidine-2'''-yle)-pipérazine-1"-yle]-butyle}-8-aza-spiro[4,5] décane-7,9-dione de la formule I pour la transformation dans l'hydrochlorure de 8-{4'-[4"-(pyrimidine-2'''-yle)-pipérazine-1"-yle]-butyle}-8-aza-spiro[4,5]décane-7,9-dione dans la forme de la solution contenant la base de 8-{4'-[4"-(pyrimidine-2'''-yle)pipérazine-1"-yle]-butyle}-8-aza-spiro[4,5]décane-7,9-dione de la formule I obtenue après l'élimination du catalyseur.

12. Procédé selon les revendications 1b) ou 1c) ou 2 à 11, caractérisé en ce qu'on exécute l'adjonction de l'éthanol ou de l'isopropanol ou de l'acétate éthylique contenant du gaz hydrochlorique à la base de 8-{4'-[4"-(pyrimidine-2'''-yle)-pipérazine-1"-yle]-butyle}-8-aza-spiro[4,5]décane-7,9-dione de la formule I pour la transformation dans le hydrochlorure de 8-{4'-[4"-(pyrimidine-2'''-yle)-pipérazine-1"-yle]-butyle}-8-aza-spiro[4,5]décane-7,9-dione pendant 5 à 30 minutes.

13. Procédé selon les revendications 1b) ou 1c) ou 2 à 12, caractérisé en ce que le brassage lors du traitement de la base de 8-{4'-[4"-(pyrimidine-2'''-yle)-pipérazine-1"-yle]-butyle}-8-aza-spiro[4,5]décane-7,9-dione de la formule I dans de l'éthanol ou de l'isopropanol ou de l'acétate éthylique contenant du gaz hydrochlorique pour la préparation de l'hydrochlorure de 8-{4'-[4"-(pyrimidine-2'''-yle)-pipérazine-1"-yle]-butyle}-8-aza-spiro[4,5]décane-7,9-dione est exécuté pendant 1 à 5 heures.